(19) Europäisches Patentamt
European Patent Office
Office européen des brevets

(11) **EP 4 495 069 A1**

(12) **EUROPEAN PATENT APPLICATION**
published in accordance with Art. 153(4) EPC

(43) Date of publication:
**22.01.2025 Bulletin 2025/04**

(21) Application number: **23770865.6**

(22) Date of filing: **16.03.2023**

(51) International Patent Classification (IPC):
*C01B 33/18* [(2006.01)]          *C01F 11/46* [(2006.01)]
*A61K 8/23* [(2006.01)]          *A61K 8/25* [(2006.01)]

(52) Cooperative Patent Classification (CPC):
**A61K 8/23; A61K 8/25; C01B 33/18; C01F 11/46**

(86) International application number:
**PCT/JP2023/010212**

(87) International publication number:
**WO 2023/176912 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.03.2022 JP 2022043020**

(71) Applicant: **Sakai Chemical Industry Co., Ltd.
Sakai-shi, Osaka 590-8502 (JP)**

(72) Inventors:
• **SUEDA, Satoru**
  **Iwaki-shi, Fukushima 971-8183 (JP)**
• **KOBAYASHI, Keita**
  **Sakai-shi, Osaka 590-0985 (JP)**
• **KUNIYOSHI, Yukihiro**
  **Iwaki-shi, Fukushima 971-8183 (JP)**
• **KOMORI, Satoshi**
  **Iwaki-shi, Fukushima 971-8183 (JP)**

(74) Representative: **Cabinet Beau de Loménie
158, rue de l'Université
75340 Paris Cedex 07 (FR)**

(54) **SPHERICAL COMPOSITE PARTICLES OF BARIUM SULFATE AND SILICA, AND PRODUCTION METHOD THEREFOR**

(57)    The present invention aims to provide spherical particles satisfying the requirements concerning "hydrochloric acid-soluble matter" and "soluble barium salt" described in the standard of "Barium sulfate" in "The Japanese Standards of Quasi-Drug Ingredients 2021". The present invention relates to spherical composite particles of barium sulfate and silica with the following features: when the spherical composite particles are subjected to a purity test concerning "hydrochloric acid-soluble matter and soluble barium salt" described in the standard of "Barium sulfate" in "The Japanese Standards of Quasi-Drug Ingredients 2021", the spherical composite particles contain a hydrochloric acid-soluble matter in an amount of 15 mg or less, and a test liquid used in the test concerning "soluble barium salt" has an absorbance of less than 0.03.

## Description

### TECHNICAL FIELD

The present invention relates to spherical composite particles of barium sulfate and silica and methods for producing the spherical composite particles.

### BACKGROUND ART

Inorganic particles include particles of various shapes. Spherical inorganic particles are used in various applications such as cosmetic products and coating materials. For example, they are used for increasing the light diffusion of the applied compositions to impart a high haze or a matte finish to the compositions. Since the spherical inorganic particles easily glide on the skin, they are incorporated particularly in cosmetic products to enhance the textures such as smoothness, softness, and spreadability. Barium sulfate is used as such spherical particles because it is hardly soluble in an acid or alkali, has a low solubility in water or organic solvents, is available at a low cost, and is easily chemically synthesized. In recent years, the use of barium sulfate has been considered as an alternative material of microplastics which may cause marine pollution.

Barium sulfate in the form of composite particles with silica is suggested as spherical barium sulfate suitable for cosmetic products. Patent Literature 1 describes production of a spherical composite powder of barium sulfate and silica by preparing barium sulfate from barium hydroxide and sulfuric acid, preparing slurry containing the barium sulfate and silica sol, spray drying the slurry, and firing the dried product at 200°C to 1100°C.

### CITATION LIST

- Patent Literature

Patent Literature 1: JP 6390756 B

### SUMMARY OF INVENTION

- Technical Problem

The production method in Patent Literature 1 and similar methods are proposed as methods for producing a spherical composite powder of barium sulfate and silica. However, the spherical composite powders of barium sulfate and silica produced by such methods do not meet the requirements concerning "hydrochloric acid-soluble matter" and "soluble barium salt" described in the "Barium sulfate" part in "The Japanese Standards of Quasi-Drug Ingredients 2021" and do not satisfy "The Japanese Standards of Quasi-Drug Ingredients 2021".

In view of the current state of the art described above, the present invention aims to provide spherical particles satisfying the requirements concerning "hydrochloric acid-soluble matter" and "soluble barium salt" described in the "Barium sulfate" part in "The Japanese Standards of Quasi-Drug Ingredients 2021"

- Solution to Problem

The present inventors studied to discover a method to obtain spherical particles satisfying the requirements concerning "hydrochloric acid-soluble matter" and "soluble barium salt" described in the "Barium sulfate" part in "The Japanese Standards of Quasi-Drug Ingredients 2021". The present inventors found that spherical composite particles of barium sulfate and silica satisfying the requirements concerning "hydrochloric acid-soluble matter" and "soluble barium salt" described in the "Barium sulfate" part in "The Japanese Standards of Quasi-Drug Ingredients 2021" can be obtained by using barium sulfate prepared by a reaction between barium sulfide and sulfuric acid as a raw material, washing the barium sulfate with an aqueous solution of a sulfate of a Group 1 or Group 2 element in the periodic table or with sulfuric acid, preparing slurry using the washed barium sulfate, mixing the barium sulfate slurry with silica to give a mixture satisfying the prescribed conditions; optionally adding a salt of a Group 1 or Group 2 element in the periodic table; and spray drying and firing the resulting mixture. Based on the finding, the present invention has been completed.

Specifically, the present invention relates to spherical composite particles of barium sulfate and silica with the following features:

when the spherical composite particles are subjected to a purity test concerning "hydrochloric acid-soluble matter and soluble barium salt" described in the standard of "Barium sulfate" in "The Japanese Standards of Quasi-Drug Ingredients 2021", the spherical composite particles contain a hydrochloric acid-soluble matter in an amount of 15 mg or less, and

a test liquid used in the test concerning "soluble barium salt" has an absorbance of less than 0.03.

The spherical composite particles of barium sulfate and silica preferably satisfy the standard of "Barium sulfate" in "The Japanese Standards of Quasi-Drug Ingredients 2021".

The spherical composite particles of barium sulfate and silica preferably have a sphericity of 1.10 or less.

The present invention also relates to a cosmetic product containing the spherical composite particles of barium sulfate and silica of the present invention.

The present invention also relates to a method for producing spherical composite particles of barium sulfate and silica, the production method including:

a first step of washing barium sulfate obtained by a reaction between barium sulfide and sulfuric acid with an aqueous solution of a sulfate of a Group 1 or Group 2 element in the periodic table or with sulfuric acid,

a second step of repulping the washed barium sulfate in a solvent to prepare barium sulfate slurry,

a third step involving: mixing the barium sulfate slurry with silica to give a mixture; spray drying a portion of the mixture; firing the dried mixture at 800°C to 1100°C; and subjecting the fired product to a purity test concerning "hydrochloric acid-soluble matter and soluble barium salt" described in the standard of "Barium sulfate" in "The Japanese Standards of Quasi-Drug Ingredients 2021" to obtain a mixture having the following feature (a) or (b),

(a) the mixture contains a hydrochloric acid-soluble matter in an amount of 15 mg or less, and a test liquid used in the test concerning "soluble barium salt" has an absorbance of less than 0.03, or

(b) the mixture contains a hydrochloric acid-soluble matter in an amount of 14 mg or less, and a test liquid used in the test concerning "soluble barium salt" has an absorbance of 0.03 or more;

a fourth step, which is performed when the mixture obtained in the third step has the feature (b), of adding a salt of a Group 1 or Group 2 element in the periodic table to the mixture at a percentage of 0.05 to 0.40% by mass relative to the weight of the raw material barium sulfate;

a fifth step of spray drying the mixture having undergone or not undergone the addition of a salt of a Group 1 or Group 2 element in the periodic table to obtain a dried product, and

a sixth step of firing the dried product at 800°C to 1100°C.

The mixing the barium sulfate slurry with silica in the third step is preferably performed while mixing the silica such that the weight percentage of the silica is 1% or more in the total weight of the barium sulfate and the silica in the barium sulfate slurry.

The silica preferably has an average particle size of 1 to 100 nm.

- Advantageous Effects of Invention

The spherical composite particles of barium sulfate and silica of the present invention satisfy the requirements concerning "hydrochloric acid-soluble matter" and "soluble barium salt" described in the "Barium sulfate" part in "The Japanese Standards of Quasi-Drug Ingredients 2021". Such spherical composite particles can suitably be used as raw materials of cosmetic products and other products which need to satisfy strict impurity content requirements.

BRIEF DESCRIPTION OF DRAWINGS

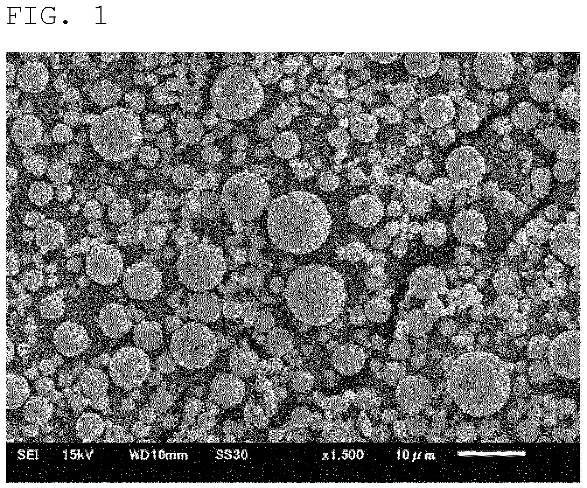

FIG. 1 shows a scanning electron microscopic image of spherical composite particles of barium sulfate and silica produced in Example 1.

DESCRIPTION OF EMBODIMENTS

A preferred embodiment of the present invention is specifically described below. Yet, the present invention is not limited to the following description, and modification may be suitably made without departing from the gist of the present invention.

1. Spherical composite particles of barium sulfate and silica

The spherical composite particles of barium sulfate and silica of the present invention have the following features:

when the spherical composite particles are subjected to a purity test concerning "hydrochloric acid-soluble matter and soluble barium salt" described in the standard of "Barium sulfate" in "The Japanese Standards of Quasi-Drug Ingredients 2021", the spherical composite particles contain a hydrochloric acid-soluble matter in an amount of 15 mg or less, and a test liquid used in the test concerning "soluble barium salt" has an absorbance of less than 0.03. Thus, the spherical composite particles can suitably be used as raw materials of cosmetic products and other products which need to satisfy strict impurity content requirements.

When analyzed by a purity test concerning "hydrochloric acid-soluble matter and soluble barium salt", the spherical composite particles contain a hydrochloric acid-soluble matter in an amount of preferably 14 mg or less, more preferably 13 mg or less.

A test liquid used in the test concerning "soluble barium salt" preferably has an absorbance of 0.025 or less, more preferably 0.020 or less.

The spherical composite particles of barium sulfate and silica of the present invention preferably satisfy the standard of "Barium sulfate" in "The Japanese Standards of Quasi-Drug Ingredients 2021". The spherical composite particles satisfying the standard can be used as raw materials of various products classified into quasi-pharmaceutical products as well as cosmetic products.

The spherical composite particles of barium sulfate and silica preferably have a sphericity of 1.10 or less. When the spherical composite particles having a sphericity of 1.10 or less are used as raw materials of cosmetic products, the cosmetic products can have better textures such as smoothness, softness, and spreadability.

The sphericity of the spherical composite particles of barium sulfate and silica is more preferably 1.09 or less, still more preferably 1.08 or less.

The sphericity of the spherical composite particles of barium sulfate and silica can be measured by the method described later in EXAMPLES.

The spherical composite particles of barium sulfate and silica of the present invention has a silica content of 1% by mass or more, preferably 3 to 20% by mass relative to the entire spherical composite particles of barium sulfate and silica. The spherical composite particles having such a silica content can have a higher sphericity to be closer to true sphere without losing the properties of barium sulfate. The spherical composite particles of barium sulfate and silica have a silica content of more preferably 5 to 14% by mass, still more preferably 7 to 10% by mass relative to the entire spherical composite particles of barium sulfate and silica.

The spherical composite particles of barium sulfate and silica of the present invention preferably have an average particle size of 2.5 to 15 um. The spherical composite particles having such an average particle size can suitably be used as raw materials of cosmetic products, etc. The average particle size is more preferably 3.0 to 10 um, still more preferably 3.5 to 8 um.

The average particle size of the spherical composite particles of barium sulfate and silica can be measured by the method described later in EXAMPLES.

The spherical composite particles also preferably contain a layer that is surface-treated with a water-repellent organic compound. The surface treatment with a water-repellent organic compound refers to water repellent treatment for reducing the affinity of the surfaces of the spherical composite particles to water.

Examples of the surface treatment with a water-repellent organic compound include organic surface treatment with an organic silicon compound, an organic aluminum compound, an organic titanium compound, a higher fatty acid, a metal soap, a polyol, an alkanolamine, or the like. Two or more of these may be used for surface treatment.

Specific examples of the water-repellent organic compound include silicone oils such as hydrogen dimethicone and triethoxysilylethyl polydimethylsiloxyethyl hexyl dimethicone; alkyl silanes such as trialkoxyoctylsilane and trialkoxydecylsilane; silane coupling agents such as vinyl trialkoxysilane, 3-glycosidoxypropyltrialkoxysilane, 3-methacryloxypropylmethyldialkoxysilane, 3-aminopropyltrialkoxysilane, and 3-mercaptopropylmethyldialkoxysilane; and C12-C18 fatty acids such as stearic acid, isostearic acid, myristic acid, palmitic acid, and palm oil fatty acid and metal salts thereof. Of these, C12-C18 fatty acids and metal salts thereof are preferred.

Examples of the metal salts of the fatty acids include salts of lithium, sodium, potassium, magnesium, calcium, barium, zinc, or aluminum. Of these, salts of calcium or magnesium are particularly preferred. Preferred examples include compounds capable of forming some kinds of chemical bonds with the composite powder and may be compounds which are physically adsorbed to the composite powder.

The surface treatment with a water-repellent organic compound imparts further improved texture to the particles, and the resulting particles are blended well with oily agents when they are incorporated in cosmetic products, for example.

The amount treated with the water-repellent organic compound is preferably 0.1 to 10% by mass, more preferably 0.5 to 8% by mass relative to the spherical composite particles of barium sulfate and silica. If the treated amount is less than 0.1% by mass, the water repellency is insufficient. If the treated amount is more than 10% by mass, the cost increases and also the effects proportional to the treatment may not be obtained.

The spherical composite particles of barium sulfate and silica of the present invention have a low impurity content and satisfy the requirements concerning "hydrochloric acid-soluble matter" and "soluble barium salt" in the "Barium sulfate" part in "The Japanese Standards of Quasi-Drug Ingredients 2021". The spherical composite particles are spherical and thus easily glide on the skin. Therefore, the spherical composite particles can suitably be used as a component to improve the textures of cosmetic products such as smoothness, softness, and spreadability. A cosmetic product containing the spherical composite particles of barium sulfate and silica of the present invention is also one aspect of the present invention.

2. Method for producing spherical composite particles of barium sulfate and silica

The method for producing the spherical composite particles of barium sulfate and silica of the present invention includes

a first step of washing barium sulfate obtained by a reaction between barium sulfide and sulfuric acid with an aqueous solution of a sulfate of a Group 1 or Group 2 element in the periodic table or with sulfuric acid,
a second step of repulping the washed barium sulfate in a solvent to prepare barium sulfate slurry,
a third step involving: mixing the barium sulfate slurry with silica to give a mixture; spray drying a portion of the mixture; firing the dried mixture at 800°C to 1100°C; and subjecting the fired product to a purity test concerning "hydrochloric acid-soluble matter and soluble barium salt" described in the standard of "Barium sulfate" in "The Japanese Standards of Quasi-Drug Ingredients 2021" to obtain a mixture having the following feature (a) or (b),

(a) the mixture contains a hydrochloric acid-soluble matter in an amount of 15 mg or less, and a test liquid used in the test concerning "soluble barium salt" has an absorbance of less than 0.03, or
(b) the mixture contains a hydrochloric acid-soluble matter in an amount of 14 mg or less, and a test liquid used in the test concerning "soluble barium salt" has an absorbance of 0.03 or more;

a fourth step, which is performed when the mixture obtained in the third step has the feature (b), of adding a salt of a Group 1 or Group 2 element in the periodic table to the mixture at a percentage of 0.05 to 0.40% by mass relative to the weight of the raw material barium sulfate;
a fifth step of spray drying the mixture having undergone or not undergone the addition of a salt of a Group 1 or Group 2 element in the periodic table to obtain a dried product, and
a sixth step of firing the dried product at 800°C to 1100°C.

Barium sulfate may be produced by, for example, reacting barium hydroxide with sulfuric acid, reacting barium chloride with sodium sulfate, or reacting barium sulfide with sulfuric acid.

With regard to the barium sulfate used as a raw material in the production of spherical composite particles of barium sulfate and silica, the present inventors discovered one of the causes which prevent the spherical composite particles of barium sulfate and silica from satisfying the requirements concerning "hydrochloric acid-soluble matter" and "soluble barium salt" in the standard of "Barium sulfate" in "The Japanese Standards of Quasi-Drug Ingredients 2021". Specifically, the cause is barium carbonate formed in a reaction between a portion of barium hydroxide with carbon dioxide in the air in the case where the barium sulfate is prepared by reacting barium hydroxide with sulfuric acid, or unreacted sodium sulfate and sodium chloride formed in the case where the barium sulfate is prepared by reacting barium chloride with sodium sulfate. Thus, the method for producing the spherical composite particles of barium sulfate and silica of the present invention uses, as a raw material, barium sulfate obtained by reacting barium sulfide with sulfuric acid.

The barium sulfate used as a raw material in the method for producing the spherical composite particles of barium sulfate and silica preferably has a pH of 9.0 or less when it is in a dry state. The barium sulfate having a pH of more than 9.0 may increase a hydrochloric acid-soluble matter contained in the resulting spherical composite particles of barium sulfate and silica. The raw material barium sulfate in a dry state has a pH of more preferably 8.8 or less, still more preferably 8.6 or less.

The pH of the raw material barium sulfate in a dry state can be measured in accordance with "methods for pigments, pH value, hot extraction method" described in JIS K 5101.

The barium sulfate used as a raw material in the method for producing the spherical composite particles of barium sulfate and silica preferably has an average particle size of 0.005 to 0.25 um. When the barium sulfate with such a particle size is used, the resulting spherical composite particles of barium sulfate and silica are more suitable for use in cosmetic products. The average particle size of the barium sulfate used as a raw material is more preferably 0.01 to 0.15 um, still more preferably 0.02 to 0.08 $\mu$m.

The average particle size of the barium sulfate used as a raw material can be measured by the method described later in EXAMPLES.

The first step involves washing barium sulfate as a raw material with an aqueous solution of a sulfate of a Group 1 or Group 2 element in the periodic table or with sulfuric acid. The washing converts a barium salt generated a little as a by-product impurity in the preparation of the barium sulfate as a raw material into barium sulfate, whereby a water soluble barium salt or an acid soluble barium salt can be removed.

The washing may be performed using either of an aqueous solution of a sulfate of a Group 1 element in the periodic table, an aqueous solution of a sulfate of a Group 2 element in the periodic table, or sulfuric acid. The washing may be performed only once or multiple times. In multiple times of washing, two or more of an aqueous solution of a sulfate of a Group 1 element in the periodic table, an aqueous solution of a sulfate of a Group 2 element in the periodic table, and sulfuric acid may be used.

The concentration of the aqueous solution of a sulfate of a Group 1 or Group 2 element in the periodic table or sulfuric acid used in the washing is not limited as long as impurities are removed from the barium sulfate. Still, the concentration is preferably 0.001 to 0.5 mol/L. With such a concentration, even a small amount of the aqueous solution or sulfuric acid can sufficiently remove impurities from the barium sulfate. The concentration of the aqueous solution of a sulfate of a Group 1 or Group 2 element in the periodic table or sulfuric acid is more preferably 0.001 to 0.1 mol/L, still more preferably 0.001 to 0.01 mol/L.

In the first step, the percentage of the aqueous solution of a sulfate of a Group 1 or Group 2 element in the periodic table or sulfuric acid used per washing is preferably 0.01 to 10% by mass relative to 100% by mass of the barium sulfate to be washed. The use of the aqueous solution of a sulfate of a Group 1 or Group 2 element in the periodic table or dilute sulfuric acid at the above-described percentage can sufficiently wash the barium sulfate while reducing the amount of the aqueous solution of a sulfate of a Group 1 or Group 2 element in the periodic table or sulfuric acid used. The percentage of the aqueous solution of a sulfate of a Group 1 or Group 2 element in the periodic table or sulfuric acid used per washing is more preferably 0.1 to 5% by mass, still more preferably 0.5 to 1% by mass relative to 100% by mass of the barium sulfate to be washed.

In the first step, the washing of the barium sulfate as a raw material with the aqueous solution of a sulfate of a Group 1 or Group 2 element in the periodic table or sulfuric acid is preferably performed at a temperature of 5°C to 100°C. When the washing is performed at such temperature, water soluble barium salts, which are impurities, can be sufficiently removed. The washing may be more preferably performed at 10°C to 100°C, still more preferably performed at 20°C to 100°C.

The duration of the washing is preferably 5 to 120 minutes. When the duration of the washing is within this range, spherical composite particles of barium sulfate and silica can be efficiently produced while sufficiently removing water soluble barium salts. The duration of the washing is more preferably 5 to 60 minutes, still more preferably 10 to 30 minutes. In multiple times of washing, the total duration of the multiple times of washing is preferably within the range indicated above.

In the first step, the washing may be performed by washing solid barium sulfate with the aqueous solution of a sulfate of a Group 1 or Group 2 element in the periodic table or sulfuric acid added thereto or by washing barium sulfate slurry with the aqueous solution of a sulfate of a Group 1 or Group 2 element in the periodic table or sulfuric acid added thereto. Preferably, the washing is performed by washing barium sulfate slurry with the aqueous solution of a sulfate of a Group 1 or Group 2 element in the periodic table or sulfuric acid added thereto, thereby enhancing the effect of washing the barium sulfate and more efficiently removing impurities such as water soluble barium salts or acid soluble barium salts.

Examples of the solvent used in the preparation of the barium sulfate slurry include one or two or more of water and alcohols. Water is preferred among these.

The concentration of the barium sulfate slurry is not limited. Still, the concentration is preferably 20 to 200 g/L to increase the effect of washing the barium sulfate while maintaining productivity. The concentration is more preferably 40 to 150 g/L, still more preferably 60 to 100 g/L.

Examples of the aqueous solution of a sulfate of a Group 1 or Group 2 element in the periodic table used in the first step include sodium sulfate, potassium sulfate, magnesium sulfate, and calcium sulfate. One or two or more of these may suitably be used.

After the washing in the first step and before the second step, the barium sulfate may be washed with water. Specifically, the barium sulfate slurry washed in the first step is filtered, and a cake obtained by the filtration is washed with water. In this case, the water washing is preferably performed until the electric conductivity of the water used in the washing reaches 200 μS/cm or less. Thus, water soluble impurities contained in the barium sulfate can be sufficiently removed. The washing is more preferably performed until the electric conductivity of the water used in the washing reaches 100 μS/cm or less and still more preferably performed until the electric conductivity of the water used in the washing reaches 50 μS/cm or less.

The second step involves repulping the washed barium sulfate in a solvent to prepare barium sulfate slurry.

Examples of the solvent used in the second step include one or two or more of water, monohydric alcohols, and polyhydric alcohols.

Any one of water and monohydric alcohols is preferred among these, with water being more preferred.

The concentration of the barium sulfate slurry prepared by repulping in the second step is not limited. Still, the concentration is preferably 50 to 1000 g/L, more preferably 200 to 900 g/L, still more preferably 300 to 800 g/L.

The third step involves: mixing the barium sulfate slurry prepared in the second step with silica to give a mixture; spray drying a portion of the mixture; firing the dried mixture at 800°C to 1100°C; and subjecting the fired product to a purity test concerning "hydrochloric acid-soluble matter and soluble barium salt" described in the standard of "Barium sulfate" in "The Japanese Standards of Quasi-Drug Ingredients 2021" to obtain a mixture having the following feature (a) or (b),

(a) the mixture contains a hydrochloric acid-soluble matter in an amount of 15 mg or less, and a test liquid used in the test concerning "soluble barium salt" has an absorbance of less than 0.03, or
(b) the mixture contains a hydrochloric acid-soluble matter in an amount of 14 mg or less, and a test liquid used in the test concerning "soluble barium salt" has an absorbance of 0.03 or more.

When the barium sulfate slurry and silica are mixed and then fired, a trace of barium silicate may be generated as a by-product. Barium silicate reacts with hydrochloric acid to generate barium chloride corresponding to "soluble barium salt". If a large amount of barium silicate is generated as a by-product, the requirements concerning "soluble barium salt" described in the standard of "Barium sulfate" in "The Japanese Standards of Quasi-Drug Ingredients 2021" are not satisfied.

In the case where a product obtained by spray drying a portion of the mixture obtained in the third step and firing the dried mixture at 800°C to 1100°C has the feature (a), spherical composite particles of barium sulfate and silica obtained by spray drying the mixture and firing the dried mixture at 800°C to 1100°C can have the following feature:
when the spherical composite particles are subjected to a purity test concerning "hydrochloric acid-soluble matter and soluble barium salt" described in the standard of "Barium sulfate" in "The Japanese Standards of Quasi-Drug Ingredients 2021", the spherical composite particles contain a hydrochloric acid-soluble matter in an amount of 15 mg or less, and a test liquid used in the test concerning "soluble barium salt" has an absorbance of less than 0.03.

In the case where a product obtained by spray drying a portion of the mixture obtained in the third step and firing the dried mixture at 800°C to 1100°C has the feature (b), the desired spherical composite particles of barium sulfate and silica cannot be obtained only by spray drying the mixture and firing the dried mixture. Yet, when a salt of a Group 1 or Group 2 element in the periodic table is added to the mixture to convert soluble barium salts contained a little as impurities in the mixture into barium sulfate in advance to reduce the amount of barium silicate generated as a by-product in the firing at 800°C to 1100°C, the amount of barium chloride generated in a test concerning "hydrochloric acid-soluble matter" described in "The Japanese Standards of Quasi-Drug Ingredients 2021" can be reduced, thereby satisfying the standard of "soluble barium salt". The salt of a Group 1 or Group 2 element in the periodic table corresponds to a hydrochloric acid-soluble matter. The mixture containing 14 mg or less of a hydrochloric acid-soluble matter has a room to safely satisfy the standard concerning hydrochloric acid-soluble matter even if a salt of a Group 1 or Group 2 element in the periodic table is added thereto. Thus, both of the standards concerning "hydrochloric acid-soluble matter" and "soluble barium salt" can be satisfied by the addition of a salt of a Group 1 or Group 2 element in the periodic table.

If the mixture obtained in the third step has none of the feature (a) and the feature (b), the procedure to the third step is performed again while using different raw materials, for example.

In the third step involving mixing the barium sulfate slurry prepared in the second step with silica to give a mixture and spray drying a portion of the mixture and firing the dried mixture, the temperature and duration of the drying are preferably the same as the temperature and duration of the drying in the fifth step described later, and the temperature and duration of the firing are preferably the same as the temperature and duration of the firing of a dried product obtained in the fifth step performed in the sixth step described later. The mixture is spray dried and fired under the same spray drying conditions as in the fifth step and the same firing conditions as in the sixth step to determine whether the mixture has the feature (a) or the feature (b). Whereby, it is possible to more reliably obtain spherical composite particles of barium sulfate and silica with the following features: when the spherical composite particles are subjected to a purity test concerning "hydrochloric acid-soluble matter and soluble barium salt" described in the standard of "Barium sulfate" in "The Japanese Standards of Quasi-Drug Ingredients 2021", the spherical composite particles contain a hydrochloric acid-soluble matter in an amount of 15 mg or less, and a test liquid used in the test concerning "soluble barium salt" has an absorbance of less than 0.03.

In the third step, the mixing of silica is preferably performed such that the weight percentage of the silica is 1% or more in the total weight of the barium sulfate and the silica in the barium sulfate slurry. The silica has an effect of allowing the composite particles of barium sulfate and silica to have a beautiful spherical shape and also has an effect of reducing the viscosity of the barium sulfate slurry. When the percentage of the silica is controlled as described above in the third step, the viscosity of the barium sulfate slurry is reduced, so that the barium sulfate slurry is more suitably dried by spray drying in the fifth step.

The percentage of the silica mixed in the third step is more preferably controlled such that the weight percentage of the silica is 3% or more, still more preferably 5% or more, particularly preferably 7% or more in the total weight of the barium sulfate and the silica in the barium sulfate slurry.

To synthesize particles satisfying the requirement concerning "hydrochloric acid-soluble matter" described in the standard of "Barium sulfate" in "The Japanese Standards of Quasi-Drug Ingredients 2021", the percentage of the silica mixed in the third step is preferably controlled such that the weight percentage of the silica is 20% or less, more preferably 14% or less, still more preferably 10% or less in the total weight of the barium sulfate and the silica in the barium sulfate slurry.

The silica to be mixed with the barium sulfate slurry in the third step preferably has an average particle size of 1 to 100 nm. The use of silica having a small particle size further reduces the viscosity of the barium sulfate slurry, so that the barium sulfate slurry is more suitably dried by spray drying in the fifth step. The use of silica having a small particle size also allows the finally obtained composite particles of the barium sulfate and the silica to have beautiful surfaces with few projections and depressions. In contrast, silica having a large particle size has a small specific surface area and thus has fewer contacting points with the barium sulfate. Thus, the amount of barium silicate generated by mixing the silica with the barium sulfate slurry tends to decrease. Taking the above into consideration, the silica used in the third step preferably has an average particle size of 1 to 100 nm.

The average particle size of the silica is more preferably 1 to 60 nm, still more preferably 1 to 20 nm. The average particle size of the silica can be measured according to the method described later in EXAMPLES.

The silica to be added to the barium sulfate slurry in the third step may be solid silica or silica sol obtained by dispersing silica in a solvent. Silica sol is preferred among these because silica particles are well dispersed in advance and no operation to separate aggregated silica particles, such as wet media dispersion treatment, is necessary. Examples of the solvent in the silica sol include the solvents used in the preparation of the barium sulfate slurry described above.

The silica to be mixed with the barium sulfate slurry in the third step may form acidic silica sol, alkaline silica sol, or neutral silica sol when dispersed in water.

The fourth step involves adding a salt of a Group 1 or Group 2 element in the periodic table to the mixture having the feature (b) at a percentage of 0.05 to 0.40% by mass relative to the weight of the raw material barium sulfate to satisfy both the standards concerning "hydrochloric acid-soluble matter" and "soluble barium salt".

Both the standards concerning "hydrochloric acid-soluble matter" and "soluble barium salt" described above can be satisfied by adding a salt of a Group 1 or Group 2 element in the periodic table to the mixture having the feature (b) at a percentage described above.

The amount of the salt of a Group 1 or Group 2 element in the periodic table to be added in the fourth step is more preferably 0.07 to 0.40% by mass, still more preferably 0.10 to 0.35% by mass relative to 100% by mass of the barium sulfate used as a raw material in the method for producing the spherical composite particles of barium sulfate and silica of the present invention.

Examples of the Group 1 or Group 2 element in the periodic table constituting the salt of a Group 1 or Group 2 element in the periodic table in the fourth step include sodium, potassium, magnesium, and calcium. A Group 1 element in the periodic table is preferred among these.

The salt of a Group 1 or Group 2 element in the periodic table is preferably a sulfate.

The fifth step involves spray drying the mixture having undergone or not undergone the addition of a salt of a Group 1 or Group 2 element in the periodic table in the fourth step to obtain a dried product.

The drying temperature is not limited as long as the mixture is dried. Taking sufficient drying of the mixture and production efficiency into consideration, the drying temperature is preferably 100°C to 250°C, more preferably 100°C to 230°C, still more preferably 105°C to 220°C.

The sixth step involves firing the dried product obtained in the fifth step at 800°C to 1100°C. The firing at such temperature can sufficiently remove sulfide contained a little in the barium sulfate and can also sufficiently fire the mixture of barium sulfate and silica into stronger particles while preventing excessive sintering of the barium sulfate.

The firing temperature is 800°C to 1100°C, preferably 800°C to 1000°C, more preferably 850°C to 950°C.

Duration of the firing (time period of maintaining the maximum temperature from when reaching the maximum temperature) is not limited. In view of the productivity, the duration is preferably 10 to 1500 minutes, more preferably 10 to 600 minutes, still more preferably 30 to 300 minutes, particularly preferably 60 to 150 minutes, most preferably 60 to 120 minutes.

The method for producing the spherical composite particles of barium sulfate and silica of the present invention may include additional steps other than the first step to the sixth step and the above-described water washing step between the first step and the second step. Examples of the additional steps include a step of dispersing the slurry prepared by mixing the barium sulfate slurry obtained in the third step with silica using a wet media disperser or a wet media-less disperser and a step of passing the slurry prepared by mixing the barium sulfate slurry obtained in the third step with silica through a sieve with an aperture of 20 to 250 um.

EXAMPLES

Specific examples are given below to describe the present invention in detail; however, the present invention is not limited to the examples. Hereinbelow, "%" refers to "% by mass (% by weight)" unless otherwise stated. The physical properties are measured by the methods described below.

<Absorbance>

Absorbance was measured using a U-1900 ratio beam spectrophotometer (Hitachi High-Technologies Corporation) at a wavelength of 500 nm and an optical path length of 20 mm.

<Average particle sizes of raw material barium sulfate and silica sol (silica)>

The primary particle size of the raw material barium sulfate or the silica broadly corresponds to the diameter of a sphere having a surface area that is the same as the specific surface area of the raw material barium sulfate or the silica determined by the BET method. Thus, the specific surface areas: Sg of the raw material barium sulfate and the silica were determined by the BET method using a full automatic BET specific surface area analyzer "Macsorb" (Mountech). The determined values were each substituted into the following equation to determine the average particle size.

$$\text{Average particle size (µm)} = 6/(\text{Sg} \times \rho)$$

Sg ($m^2$/g): Specific surface area
$\rho$ (g/$cm^3$): particle density
The density: $\rho$ (g/$cm^3$) of the barium sulfate, 4.5 (g/$cm^3$) was used. While 2.2 (g/$cm^3$) was used for the density of the silica.

<Average particle size of composite particles of barium sulfate and silica>

The average particle size (D50) was measured with a laser diffraction-scattering particle size distribution analyzer "Microtrac MT3300EXII" (Nikkiso Co., Ltd.). Water was used as a solvent.

<Sphericity>

Diagonal lines were drawn on an image taken with a scanning electron microscope "JSM-6510A" (Jeol Ltd.). The long diameters and the short diameters of 50 particles on the diagonal lines were measured. An average of the ratios: long diameter/short diameter of the particles was defined as the sphericity.

Example 1

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 135 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, stirring for 30 minutes, filtration, and water washing until the electric conductivity reached 40 µS/cm or less. The solid component remaining after the water washing was repulped in pure water to allow the slurry to have a concertation of 380 g/L. To the slurry was added silica sol (SNOWTEX ST-30, Nissan Chemical Industries, Ltd.) such that the weight ratio of $BaSO_4$ and $SiO_2$ was 93:7, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 15 mg or less (8.0 mg) and a test liquid used in a test concerning "soluble barium salt" had an absorbance of less than 0.03 (0.004).

Therefore, the mixture was directly spray dried with a micromist spray drier (MDL-050CM, GF). The dried mixture was fired at 900°C for two hours to prepare 3.5 um spherical composite particles of barium sulfate and silica. The particles had a sphericity of 1.03. FIG. 1 shows a scanning electron microscopic image of the spherical composite particles of barium sulfate and silica.

The powder of the spherical composite particles of barium sulfate and silica was subjected to a purity test of "barium sulfate" described in the standard of "Barium sulfate" in "The Japanese Standards of Quasi-Drug Ingredients 2021". The result demonstrated that the powder met all the following standard items and therefore satisfied "The Japanese Standards of Quasi-Drug Ingredients 2021". The amount of hydrochloric acid-soluble matter was 8.0 mg and a test liquid after the analysis concerning "soluble barium salt" had an absorbance of 0.004.

· Phosphate: not detected
· Sulfide: not detected
· Hydrochloric acid-soluble matter: 15 mg or less (8.0 mg)
· Soluble barium salt: not detected
· pH: neutral
· Heavy metal: 10 ppm or less
· Arsenic: 1 ppm or less

The purity test of "barium sulfate" described in the standard of "Barium sulfate" in "The Japanese Standards of Quasi-Drug Ingredients 2021" is as follows.

Purity test

(1) pH: A liquid prepared by adding 20 mL of water to 1.0 g of sample and shaking for five minutes is neutral.
(2) Phosphate: To 1.0 g of sample is added 8 mL of diluted (3 → 8) nitric acid and subsequently boiled for five minutes. After cooling, water is supplied to make up the evaporated water to the original volume. The resulting mixture is filtered through a filter paper washed with dilute nitric acid. To the filtrate is added an equal volume of a hexaammonium heptamolybdate reagent and then left at 50°C to 60°C for one hour. As a result, no yellow precipitate is generated.
(3) Sulfide: To 10 g of sample is added 10 mL of dilute hydrochloric acid and water to a volume of 100 mL to prepare a sample solution. Gas generated during boiling of the sample solution for 10 minutes does not turn a wet lead acetate (II) sheet black.
(4) Hydrochloric acid-soluble matter and soluble barium salt: The sample solution in the above (3) is cooled, and then water is added thereto to a volume of 100 mL, followed by filtration. Next, 50 mL of the filtrate is dried and solidified by evaporation on a water bath. To the resulting product are added two drops of hydrochloric acid and 10 mL of warm water, followed by filtration through a filter paper for quantitative analysis which is dried at 105°C for one hour in advance. The residue is washed with 100 mL of warm water. The liquid after the washing is combined with the filtrate and then evaporated to dryness and solidified on a water bath. When the residue is dried at 105°C for one hour, the amount of the resulting product is 15 mg or less. Moreover, when a liquid is prepared by adding 10 mL of water to the product, followed by mixing by shaking, filtration, adding 0.5 mL of dilute sulfuric acid to the filtrate, and leaving for 30 minutes, the liquid does not become turbid.
(5) Heavy metal: To 5.0 g of sample were added 2.5 mL of acetic acid (100) and 50 mL of water and subsequently boiled for 10 minutes. After cooling, 0.5 mL of an ammonia reagent and water are added to a volume of 100 mL, followed by filtration. When the test is performed using 50 mL of the filtrate as a sample solution, the upper limit of the heavy metal content is 10 ppm or less. Here, 2.5 mL of a lead standard liquid is used as a comparative liquid.
(6) Arsenic: To 2.0 g of sample was added 10 mL of dilute sulfuric acid and then warmed to prepare a sample solution. When the test is performed using the sample solution, the upper limit of the arsenic content is 1 ppm or less.

Example 2

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 135 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, stirring for 30 minutes, filtration, and water washing until the electric conductivity reached 40 μS/cm or less. The solid component remaining after the water washing was repulped in pure water to allow the slurry to have a concertation of 380 g/L. To the slurry was added silica sol (SNOWTEX ST-O, Nissan Chemical Industries, Ltd.) such that the weight ratio of $BaSO_4$ and $SiO_2$ was 90:10, and mixed together.
A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 14 mg or less (7.9 mg) and a test liquid after a test concerning "soluble barium salt" had an absorbance of 0.03 or more (0.925).
Therefore, sodium sulfate (Kishida Chemical Co., Ltd.) was added to the obtained mixture in an amount of 2500 ppm relative to the weight of the barium sulfate and stirred for 10 minutes. Thereafter, the mixture was spray dried with a micromist spray drier (MDL-050CM, GF). The dried mixture was fired at 900°C for two hours to prepare 3.5 um spherical composite particles of barium sulfate and silica. The particles had a sphericity of 1.03.
The powder of the spherical composite particles of barium sulfate and silica was subjected to a test of "barium sulfate" described in "The Japanese Standards of Quasi-Drug Ingredients 2021". The result demonstrated that the powder met all the following standard items and therefore satisfied the standard of "Barium sulfate" in "The Japanese Standards of Quasi-Drug Ingredients 2021". The amount of hydrochloric acid-soluble matter was 12.5 mg and a test liquid after the analysis concerning "soluble barium salt" had an absorbance of 0.003.

· Phosphate: not detected
· Sulfide: not detected
· Hydrochloric acid-soluble matter: 15 mg or less (12.5 mg)
· Soluble barium salt: not detected
· pH: neutral
· Heavy metal: 10 ppm or less
· Arsenic: 1 ppm or less

Example 3

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 135 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, increasing the temperature to 100°C and stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-30, Nissan Chemical Industries, Ltd.) such that the weight ratio of $BaSO_4$ and $SiO_2$ was 93:7, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 15 mg or less (7.2 mg) and a test liquid after a test concerning "soluble barium salt" had an absorbance of less than 0.03 (0.007).

Therefore, the obtained mixture was directly spray dried with a micromist spray drier (MDL-050CM, GF). The dried mixture was fired at 800°C for two hours to prepare 3.5 um spherical composite particles of barium sulfate and silica. The particles had a sphericity of 1.04.

The powder of the spherical composite particles of barium sulfate and silica was subjected to a test of "barium sulfate" described in "The Japanese Standards of Quasi-Drug Ingredients 2021". The result demonstrated that the powder met all the following standard items and therefore satisfied "The Japanese Standards of Quasi-Drug Ingredients 2021". The amount of hydrochloric acid-soluble matter was 7.2 mg and a test liquid after the analysis concerning "soluble barium salt" had an absorbance of 0.007.

· Phosphate: not detected
· Sulfide: not detected
· Hydrochloric acid-soluble matter: 15 mg or less (7.2 mg)
· Soluble barium salt: not detected
· pH: neutral
· Heavy metal: 10 ppm or less
· Arsenic: 1 ppm or less

Example 4

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 90 g/L by adding pure water thereto. To the slurry of the raw material barium sulfate was added sulfuric acid in an amount of 0.8% relative to the barium sulfate, followed by stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-30, Nissan Chemical Industries, Ltd.) such that the weight ratio of $BaSO_4$ and $SiO_2$ was 93:7, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 15 mg or less (10.5 mg) and a test liquid after a test concerning "soluble barium salt" had an absorbance of less than 0.03 (0.008).

Therefore, the obtained mixture was directly spray dried with a micromist spray drier (MDL-050CM, GF). The dried mixture was fired at 900°C for two hours to prepare 3.5 um spherical composite particles of barium sulfate and silica. The particles had a sphericity of 1.05.

The powder of the spherical composite particles of barium sulfate and silica was subjected to a test of "barium sulfate" described in "The Japanese Standards of Quasi-Drug Ingredients 2021". The result demonstrated that the powder met all the following standard items and therefore satisfied the standard of "Barium sulfate" in "The Japanese Standards of Quasi-Drug Ingredients 2021". The amount of hydrochloric acid-soluble matter was 10.5 mg and a test liquid after the analysis concerning "soluble barium salt" had an absorbance of 0.008.

· Phosphate: not detected
· Sulfide: not detected
· Hydrochloric acid-soluble matter: 15 mg or less (10.5 mg)
· Soluble barium salt: not detected
· pH: neutral
· Heavy metal: 10 ppm or less
· Arsenic: 1 ppm or less

Example 5

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 90 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, increasing the temperature to 60°C and stirring for 30 minutes, filtration, and water washing until the electric conductivity reached 40 $\mu$S/cm or less. The solid component remaining after the water washing was repulped in pure water to allow the slurry to have a concentration of 135 g/L. To the slurry was added silica sol (SNOWTEX ST-30L, Nissan Chemical Industries, Ltd.) such that the weight ratio of $BaSO_4$ and $SiO_2$ was 85:15, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 15 mg or less (14.4 mg) and a test liquid after a test concerning "soluble barium salt" had an absorbance of less than 0.03 (0.015).

Therefore, the obtained mixture was directly spray dried with a micromist spray drier (MDL-050CM, GF). The dried mixture was fired at 900°C for two hours to prepare 3.4 um spherical composite particles of barium sulfate and silica. The particles had a sphericity of 1.07.

The powder of the spherical composite particles of barium sulfate and silica was subjected to a test of "barium sulfate" described in "The Japanese Standards of Quasi-Drug Ingredients 2021". The result demonstrated that the powder met all the following standard items and therefore satisfied the standard of "Barium sulfate" in "The Japanese Standards of Quasi-Drug Ingredients 2021". The amount of hydrochloric acid-soluble matter was 14.4 mg and a test liquid after the analysis concerning "soluble barium salt" had an absorbance of 0.015.

· Phosphate: not detected
· Sulfide: not detected
· Hydrochloric acid-soluble matter: 15 mg or less (14.4 mg)
· Soluble barium salt: not detected
· pH: neutral
· Heavy metal: 10 ppm or less
· Arsenic: 1 ppm or less

Example 6

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 90 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, increasing the temperature to 60°C and stirring for 30 minutes, filtration, and water washing until the electric conductivity reached 40 $\mu$S/cm or less. The solid component remaining after the water washing was repulped in pure water to allow the slurry to have a concentration of 135 g/L. To the slurry was added silica sol (SNOWTEX ST-OL, Nissan Chemical Industries, Ltd.) such that the weight ratio of $BaSO_4$ and $SiO_2$ was 85:15, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 15 mg or less (3.7 mg) and a test liquid after a test concerning "soluble barium salt" had an absorbance of less than 0.03 (0.008).

Therefore, the obtained mixture was directly spray dried with a micromist spray drier (MDL-050CM, GF). The dried mixture was fired at 900°C for two hours to prepare 3.6 um spherical composite particles of barium sulfate and silica. The particles had a sphericity of 1.08.

The powder of the spherical composite particles of barium sulfate and silica was subjected to a test of "barium sulfate" described in "The Japanese Standards of Quasi-Drug Ingredients 2021". The result demonstrated that the powder met all the following standard items and therefore satisfied the standard of "Barium sulfate" in "The Japanese Standards of Quasi-Drug Ingredients 2021". The amount of hydrochloric acid-soluble matter was 3.7 mg and a test liquid after the analysis concerning "soluble barium salt" had an absorbance of 0.008.

· Phosphate: not detected
· Sulfide: not detected
· Hydrochloric acid-soluble matter: 15 mg or less (3.7 mg)
· Soluble barium salt: not detected
· pH: neutral
· Heavy metal: 10 ppm or less
· Arsenic: 1 ppm or less

Example 7

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 90 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, increasing the temperature to 60°C and stirring for 30 minutes, filtration, and water washing until the electric conductivity reached 40 µS/cm or less. The solid component remaining after the water washing was repulped in pure water to allow the slurry to have a concentration of 135 g/L. To the slurry was added silica sol (0.05 um Sciqas aqueous dispersion, Sakai Chemical Industry Co., Ltd.) such that the weight ratio of $BaSO_4$ and $SiO_2$ was 85:15, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 15 mg or less (6.3 mg) and a test liquid after a test concerning "soluble barium salt" had an absorbance of less than 0.03 (0.014).

Therefore, the obtained mixture was directly spray dried with a micromist spray drier (MDL-050CM, GF). The dried mixture was fired at 900°C for two hours to prepare 3.6 um spherical composite particles of barium sulfate and silica. The particles had a sphericity of 1.07.

The powder of the spherical composite particles of barium sulfate and silica was subjected to a test of "barium sulfate" described in "The Japanese Standards of Quasi-Drug Ingredients 2021". The result demonstrated that the powder met all the following standard items and therefore satisfied the standard of "Barium sulfate" in "The Japanese Standards of Quasi-Drug Ingredients 2021". The amount of hydrochloric acid-soluble matter was 6.3 mg and a test liquid after the analysis concerning "soluble barium salt" had an absorbance of 0.014.

· Phosphate: not detected
· Sulfide: not detected
· Hydrochloric acid-soluble matter: 15 mg or less (6.3 mg)
· Soluble barium salt: not detected
· pH: neutral
· Heavy metal: 10 ppm or less
· Arsenic: 1 ppm or less

Example 8

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 135 g/L by adding pure water thereto. To the slurry of the raw material barium sulfate was added sulfuric acid in an amount of 0.8% relative to the barium sulfate, followed by stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-O, Nissan Chemical Industries, Ltd.) such that the weight ratio of $BaSO_4$ and $SiO_2$ was 90:10, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 14 mg or less (7.9 mg) and a test liquid after a test concerning "soluble barium salt" had an absorbance of 0.03 or more (0.925).

Therefore, sodium sulfate (Kishida Chemical Co., Ltd.) was added to the obtained mixture in an amount of 2500 ppm relative to the weight of the barium sulfate and stirred for 10 minutes. Thereafter, the mixture was spray dried with a micromist spray drier (MDL-050CM, GF). The dried mixture was fired at 900°C for two hours to prepare 3.5 um spherical composite particles of barium sulfate and silica.

The particles had a sphericity of 1.05. The powder of the spherical composite particles of barium sulfate and silica was subjected to a test of "barium sulfate" described in "The Japanese Standards of Quasi-Drug Ingredients 2021". The result demonstrated that the powder met all the following standard items and therefore satisfied the standard of "Barium sulfate" in "The Japanese Standards of Quasi-Drug Ingredients 2021". The amount of hydrochloric acid-soluble matter was 12.3 mg and a test liquid after the analysis concerning "soluble barium salt" had an absorbance of 0.005.

· Phosphate: not detected
· Sulfide: not detected
· Hydrochloric acid-soluble matter: 15 mg or less (12.3 mg)
· Soluble barium salt: not detected
· pH: neutral
· Heavy metal: 10 ppm or less
· Arsenic: 1 ppm or less

Comparative Example 1

Barium sulfide was reacted with sulfuric acid to prepare a raw material barium sulfate (pigment pH of 9.7 in a dry state) having an average particle size of 0.02 um. The barium sulfate was washed with water to give slurry, and the concentration of the slurry was adjusted to 90 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-O, Nissan Chemical Industries, Ltd.) such that the weight ratio of $BaSO_4$ and $SiO_2$ was 85:15, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was more than 15 mg (17.2 mg) and a test liquid after a test concerning "soluble barium salt" had an absorbance of 0.03 or more (0.936).

The obtained mixture was directly spray dried with a micromist spray drier (MDL-050CM, GF). The dried mixture was fired at 900°C for two hours to prepare 3.4 um spherical composite particles of barium sulfate and silica. The particles had a sphericity of 1.05.

The powder of the spherical composite particles of barium sulfate and silica was subjected to a test of "barium sulfate" described in "The Japanese Standards of Quasi-Drug Ingredients 2021". The result demonstrated that the amount of "hydrochloric acid-soluble matter" exceeded 15 mg, and "soluble barium salt" was detected. Therefore, the powder did not satisfy the standard of "Barium sulfate" in "The Japanese Standards of Quasi-Drug Ingredients 2021". The amount of "hydrochloric acid-soluble matter" was 17.2 mg and a test liquid after the analysis concerning "soluble barium salt" had an absorbance of 0.936.

Comparative Example 2

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 90 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-O, Nissan Chemical Industries, Ltd.) such that the weight ratio of $BaSO_4$ and $SiO_2$ was 93:7, and mixed together.
A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 14 mg or less (7.8 mg) and a test liquid after a test concerning "soluble barium salt" had an absorbance of 0.03 or more (0.853).
The obtained mixture was directly spray dried with a micromist spray drier (MDL-050CM, GF). The dried mixture was fired at 900°C for two hours to prepare 3.3 um spherical composite particles of barium sulfate and silica. The particles had a sphericity of 1.03.
The powder of the spherical composite particles of barium sulfate and silica was subjected to a test of "barium sulfate" described in "The Japanese Standards of Quasi-Drug Ingredients 2021". The result demonstrated that "soluble barium salt" was detected. Therefore, the powder did not satisfy the standard of "Barium sulfate" in "The Japanese Standards of Quasi-Drug Ingredients 2021". The amount of "hydrochloric acid-soluble matter" was 7.8 mg and a test liquid after the analysis concerning "soluble barium salt" had an absorbance of 0.853.

Comparative Example 3

Barium sulfide was reacted with sulfuric acid to prepare a raw material barium sulfate (pigment pH of 8.5 in a dry state) having an average particle size of 0.05 um. The barium sulfate was washed with water to give slurry. Washing with a washing agent was not performed. The concentration of the slurry was adjusted to 380 g/L by adding pure water thereto. To the slurry was added silica sol (SNOWTEX ST-30, Nissan Chemical Industries, Ltd.) such that the weight ratio of $BaSO_4$ and $SiO_2$ was 85:15, and mixed together.
A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was more than 15 mg (19.4 mg) and a test liquid after a test concerning "soluble barium salt" had an absorbance of less than 0.03 (0.016).
The obtained mixture was directly spray dried with a micromist spray drier (MDL-050CM, GF). The dried mixture was fired at 900°C for two hours to prepare 3.4 um spherical composite particles of barium sulfate and silica. The particles had a sphericity of 1.04.
The powder of the spherical composite particles of barium sulfate and silica was subjected to a test of "barium sulfate" described in "The Japanese Standards of Quasi-Drug Ingredients 2021". The result demonstrated that the amount of "hydrochloric acid-soluble matter" exceeded 15 mg. Therefore, the powder did not satisfy the standard of "Barium sulfate" in "The Japanese Standards of Quasi-Drug Ingredients 2021". The amount of "hydrochloric acid-soluble matter" was 19.4 mg and a test liquid after the analysis concerning "soluble barium salt" had an absorbance of 0.016.

Comparative Example 4

Barium sulfide was reacted with sulfuric acid to prepare a raw material barium sulfate (pigment pH of 8.5 in a dry state) having an average particle size of 0.05 um. The barium sulfate was washed with water to give slurry. Washing with a washing agent was not performed. The concentration of the slurry was adjusted to 380 g/L by adding pure water thereto. To the slurry was added silica sol (SNOWTEX ST-30, Nissan Chemical Industries, Ltd.) such that the weight ratio of $BaSO_4$ and $SiO_2$ was 85:15, and mixed together.
A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was more than 15 mg (21.3 mg) and a test liquid after a test concerning "soluble barium salt" had an absorbance of less than 0.03 (0.015).
The obtained mixture was directly spray dried with a micromist spray drier (MDL-050CM, GF). The dried mixture was fired at 750°C for two hours to prepare 3.4 um spherical composite particles of barium sulfate and silica. The particles had a sphericity of 1.07.

The powder of the spherical composite particles of barium sulfate and silica was subjected to a test of "barium sulfate" described in "The Japanese Standards of Quasi-Drug Ingredients 2021". The result demonstrated that "sulfide" was detected, and the amount of "hydrochloric acid-soluble matter" exceeded 15 mg. Therefore, the powder did not satisfy the standard of "Barium sulfate" in "The Japanese Standards of Quasi-Drug Ingredients 2021". The amount of "hydrochloric acid-soluble matter" was 21.3 mg and a test liquid after the analysis concerning "soluble barium salt" had an absorbance of 0.015.

Comparative Example 5

Barium chloride was reacted with sodium sulfate to prepare a raw material barium sulfate (pigment pH of 6.5 in a dry state) having an average particle size of 0.3 um. The barium sulfate was washed with water to give slurry, and the concentration of the slurry was adjusted to 135 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-30, Nissan Chemical Industries, Ltd.) such that the weight ratio of $BaSO_4$ and $SiO_2$ was 85:15, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was more than 15 mg (42.3 mg) and a test liquid after a test concerning "soluble barium salt" had an absorbance of 0.03 or more (0.032).

The obtained mixture was directly spray dried with a micromist spray drier (MDL-050CM, GF). The dried mixture was fired at 900°C for two hours to prepare 3.4 um spherical composite particles of barium sulfate and silica. The particles had a sphericity of 1.25.

The powder of the spherical composite particles of barium sulfate and silica was subjected to a test of "barium sulfate" described in "The Japanese Standards of Quasi-Drug Ingredients 2021". The result demonstrated that the amount of "hydrochloric acid-soluble matter" exceeded 15 mg, and "soluble barium salt" was detected. Therefore, the powder did not satisfy the standard of "Barium sulfate" in "The Japanese Standards of Quasi-Drug Ingredients 2021". The amount of "hydrochloric acid-soluble matter" was 42.3 mg and a test liquid after the analysis concerning "soluble barium salt" had an absorbance of 0.032.

Comparative Example 6

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 135 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-O, Nissan Chemical Industries, Ltd.) such that the weight ratio of $BaSO_4$ and $SiO_2$ was 90:10, and mixed together.

A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 14 mg or less (7.9 mg) and a test liquid after a test concerning "soluble barium salt" had an absorbance of 0.03 or more (0.925).

Sodium sulfate (Kishida Chemical Co., Ltd.) was added to the obtained mixture in an amount of 10 ppm relative to the weight of the barium sulfate and stirred for 10 minutes. Thereafter, the mixture was spray dried with a micromist spray drier (MDL-050CM, GF). The dried mixture was fired at 900°C for two hours to prepare 3.4 um spherical composite particles of barium sulfate and silica. The particles had a sphericity of 1.05.

The powder of the spherical composite particles of barium sulfate and silica was subjected to a test of "barium sulfate" described in "The Japanese Standards of Quasi-Drug Ingredients 2021". The result demonstrated that "soluble barium salt" was detected. Therefore, the powder did not satisfy the standard of "Barium sulfate" in "The Japanese Standards of Quasi-Drug Ingredients 2021". The amount of "hydrochloric acid-soluble matter" was 8.0 mg and a test liquid after the analysis concerning "soluble barium salt" had an absorbance of 0.916.

Comparative Example 7

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 135 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-O, Nissan Chemical Industries, Ltd.) such that the weight ratio of $BaSO_4$ and $SiO_2$ was 90:10, and mixed together.
A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 14 mg or less (7.9 mg) and a test liquid after a test concerning "soluble barium salt" had an absorbance of 0.03 or more (0.925).
Sodium sulfate (Kishida Chemical Co., Ltd.) was added to the obtained mixture in an amount of 100 ppm relative to the weight of the barium sulfate and stirred for 10 minutes. The resulting mixture was spray dried with a micromist spray drier (MDL-050CM, GF). The dried mixture was fired at 900°C for two hours to prepare 3.4 um spherical composite particles of barium sulfate and silica. The particles had a sphericity of 1.04.
The powder of the spherical composite particles of barium sulfate and silica was subjected to a test of "barium sulfate" described in "The Japanese Standards of Quasi-Drug Ingredients 2021". The result demonstrated that "soluble barium salt" was detected. Therefore, the powder did not satisfy the standard of "Barium sulfate" in "The Japanese Standards of Quasi-Drug Ingredients 2021". The amount of "hydrochloric acid-soluble matter" was 8.1 mg and a test liquid after the analysis concerning "soluble barium salt" had an absorbance of 0.425.

Comparative Example 8

Barium sulfide was reacted with sulfuric acid to prepare a raw material barium sulfate (pigment pH of 8.5 in a dry state) having an average particle size of 0.05 um. The barium sulfate was washed with water to give slurry, and the concentration of the slurry was adjusted to 135 g/L by adding pure water thereto. To the slurry of the raw material barium sulfate was added (anhydrous) sodium sulfate (99%, Kishida Chemical Co., Ltd.) in an amount of 0.8% relative to the barium sulfate, followed by dissolution, stirring for 30 minutes, filtration, and water washing until the electric conductivity reached 40 $\mu$S/cm or less. The solid component remaining after the water washing was repulped in pure water to allow the slurry to have a concentration of 380 g/L. To the slurry was added silica sol (SNOWTEX ST-O, Nissan Chemical Industries, Ltd.) such that the weight ratio of $BaSO_4$ and $SiO_2$ was 90:10, and mixed together.
A portion of the resulting mixture was spray dried and then fired at 900°C for two hours into a powder. The powder was subjected to a test of "barium sulfate" described in the standard of "Barium sulfate" in "The Japanese Standards of Quasi-Drug Ingredients 2021". The result of the test showed that the amount of hydrochloric acid-soluble matter was 14 mg or less (7.9 mg) and a test liquid after a test concerning "soluble barium salt" had an absorbance of 0.03 or more (0.925).
Sodium sulfate (Kishida Chemical Co., Ltd.) was added to the obtained mixture in an amount of 10000 ppm relative to the weight of the barium sulfate and stirred for 10 minutes. The resulting mixture was spray dried with a micromist spray drier (MDL-050CM, GF). The dried mixture was fired at 900°C for two hours to prepare 3.4 um spherical composite particles of barium sulfate and silica. The particles had a sphericity of 1.07.
The powder of the spherical composite particles of barium sulfate and silica was subjected to a test of "barium sulfate" described in "The Japanese Standards of Quasi-Drug Ingredients 2021". The result demonstrated that the amount of "hydrochloric acid-soluble matter" exceeded 15 mg. Therefore, the powder did not satisfy the standard of "Barium sulfate" in "The Japanese Standards of Quasi-Drug Ingredients 2021". The amount of "hydrochloric acid-soluble matter" was 27.5 mg and a test liquid after the analysis concerning "soluble barium salt" had an absorbance of 0.007.
Tables 1 and 2 summarize Examples 1 to 8 and Comparative Examples 1 to 8. The texture of the spherical composite particles of barium sulfate and silica in the tables was evaluated by the following method.

<Texture of spherical composite particles of barium sulfate and silica>

Ten panelists touched the obtained powder (sample) with the back of the hand and the index finger to evaluate the texture of the powder on a 5-point scale from 1 to 5 (5 being the best). The evaluation points were averaged. The texture was evaluated based on the following criteria. Powders "which easily glided on the skin and were smooth and light" were given 5 points. Powders "which did not glide on the skin and were not smooth" were given 1 point. ∘: Average is 3 points or more.

△: Average is less than 3 points and 2 points or more.

✕: Average is less than 2 points.

[Table 1]

| Item | | Example 1 | Example 2 | Example 3 | Example 4 | Exam ple 5 | Exam ple 6 | Example 7 | Example 8 |
|---|---|---|---|---|---|---|---|---|---|
| Raw material barium sulfate | Particle production method | Barium sulfide -Sulfuric acid | Barium sulfide -Sulfuric acid | Barium sulfide -Sulfuric acid | Barium sulfide -Sulfuric acid | Barium sulfide -Sulfuric acid | Barium sulfide -Sulfuric acid | Barium sulfide -Sulfuric acid | Barium sulfide -Sulfuric acid |
| | Average particle size ($\mu$m) | 0.05 | 0.05 | 0.05 | 0.05 | 0.05 | 0.05 | 0.05 | 0.05 |
| | Pigment pH | 8.5 | 8.5 | 8.5 | 8.5 | 8.5 | 8.5 | 8.5 | 8.5 |
| Washing conditions of raw material barium sulfate | Concentration of $BaSO_4$ to be washed (g/L) | 135 | 135 | 135 | 90 | 90 | 90 | 90 | 135 |
| | Washing agent | Sodium sulfate | Sodium sulfate | Sodium sulfate | Sulfuric acid | Sodium sulfate | Sodium sulfate | Sodium sulfate | Sulfuric acid |
| | Amount of washing agent used (to $BaSO_4$) (%) | 0.8 | 0.8 | 0.8 | 0.8 | 0.8 | 0.8 | 0.8 | 0.8 |
| | Temperature during washing (°C) | 25 | 25 | 100 | 25 | 60 | 60 | 60 | 25 |
| | Duration of washing (h) | 0.5 | 0.5 | 0.5 | 0.5 | 0.5 | 0.5 | 0.5 | 0.5 |
| Concentration of slurry upon repulping washed solid component in pure water (g/L) | | 380 | 380 | 380 | 380 | 135 | 135 | 135 | 380 |
| Silica source | Added silica sol | SNOWTEX ST-30 | SNOWTEX ST-O | SNOWTEX ST-30 | SNOWTEX ST-30 | SNOWTEX ST-30L | SNOWTEX ST-OL | 0.05 $\mu$m Sciqas aqueous dispersion | SNOWTEX ST-O |
| | Average particle size of silica ($\mu$m) | 0.012 | 0.012 | 0.012 | 0.012 | 0.045 | 0.045 | 0.05 | 0.012 |
| | pH of silica sol | Alkaline | Acidic | Alkaline | Alkaline | Alkaline | Acidic | Acidic | Acidic |
| $BaSO_4/SiO_2$ mass ratio in slurry to be spray-dried | | 93/7 | 90/10 | 93/7 | 93/7 | 85/15 | 85/15 | 85/15 | 90/10 |
| Addition of sodium sulfate | Added amount (to $BaSO_4$) (ppm) | - | 2500 | - | - | - | - | - | 2500 |

EP 4 495 069 A1

| Item | | Example 1 | Example 2 | Example 3 | Example 4 | Exam ple 5 | Exam ple 6 | Example 7 | Example 8 |
|---|---|---|---|---|---|---|---|---|---|
| Drying method | | Spray drying | Spray drying | Spray drying | Spray drying | Spray drying | Spray drying | Spray drying | Spray drying |
| Firing temperature × Duration (°C × η) | | 900 x 2 | 900 × 2 | 800 × 2 | 900 × 2 | 900 × 2 | 900 × 2 | 900 × 2 | 900 × 2 |
| Powder physical properties | Average particle size (pm) | 3.5 | 3.5 | 3.5 | 3.5 | 3.4 | 3.6 | 3.6 | 3.5 |
| | $SiO_2$ content (%) | 7 | 10 | 7 | 7 | 15 | 15 | 15 | 10 |
| | Sphericity | 1.03 | 103 | 104 | 105 | 107 | 108 | 107 | 105 |
| | Texture | ○ | ○ | ○ | ○ | △ | △ | △ | ○ |
| Result of test of "barium sulfate" in The Japanese Standards of Quasi-Drug Ingredients 2021 | Phosphate | Not detected | Not detected | Not detected | Not detected | Not detected | Not detected | Not detected | Not detected |
| | Sulfide | Not detected | Not detected | Not detected | Not detected | Not detected | Not detected | Not detected | Not detected |
| | Hydrochloric acid-soluble matter (mg) | 8.0 | 125 | 7.2 | 105 | 14.4 | 3.7 | 6.3 | 12.3 |
| | Soluble barium salt | Not detected | Not detected | Not detected | Not detected | Not detected | Not detected | Not detected | Not detected |
| | pH | Neutral | Neutral | Neutral | Neutral | Neutral | Neutral | Neutral | Neutral |
| | Heavy metal (ppm) | 10 or less | 10 or less | 10 or less | 10 or less | 10 or less | 10 or less | 10 or less | 10 or less |
| | Arsenic (ppm) | 1 or less | 1 or less | 1 or less | 1 or less | 1 or less | 1 or less | 1 or less | 1 or less |
| | Satisfied/Not satisfied | Satisfied | Satisfied | Satisfied | Satisfied | Satisfied | Satisfied | Satisfied | Satisfied |
| | Absorbance of test liquid after analysis of soluble barium salt (Wavelength 500 nm, Optical path length 20 mm) | 0.004 | 0.003 | 0.007 | 0.008 | 0.015 | 0.008 | 0.014 | 0.005 |

EP 4 495 069 A1

[Table 2]

| Item | | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|---|
| Raw material barium sulfate | Particle production method | Barium sulfide-Sulfuric acid | Barium sulfide-Sulfuric acid | Barium sulfide-Sulfuric acid | Barium sulfide-Sulfuric acid | Barium chloride-Sodium sulfate | Barium sulfide-Sulfuric acid | Barium sulfide-Sulfuric acid | Barium sulfide-Sulfuric acid |
| | Average particle size ($\mu$m) | 0.02 | 0.05 | 0.05 | 0.05 | 0.3 | 0.05 | 0.05 | 0.05 |
| | Pigment pH | 9.7 | 8.5 | 8.5 | 8.5 | 6.5 | 8.5 | 8.5 | 8.5 |
| Washing conditions of raw material barium sulfate | Concentration of $BaSO_4$ to be washed (g/L) | 90 | 90 | - | - | 135 | 135 | 135 | 135 |
| | Washing agent | Sodium sulfate | Sodium sulfate | - | - | Sodium sulfate | Sodium sulfate | Sodium sulfate | Sodium sulfate |
| | Amount of washing agent used (to $BaSO_4$) (%) | 0.8 | 0.8 | - | - | 0.8 | 0.8 | 0.8 | 0.8 |
| | Temperature during washing (°C) | 25 | 25 | - | - | 25 | 25 | 25 | 25 |
| | Duration of washing (h) | 0.5 | 0.5 | - | - | 0.5 | 0.5 | 0.5 | 0.5 |
| Concentration of slurry upon repulping washed solid component in pure water (g/L) | | 380 | 380 | 380 | 380 | 380 | 380 | 380 | 380 |
| Silica source | Added silica sol | SNOWTEX ST-O | SNOWTEX ST-O | SNOWTEX ST-30 | SNOWTEX ST-30 | SNOWTEX ST-30 | SNOWTEX ST-O | SNOWTEX ST-O | SNOWTEX ST-O |
| | Average particle size of silica ($\mu$m) | 0.012 | 0.012 | 0.012 | 0.012 | 0.012 | 0.012 | 0.012 | 0.012 |
| | pH of silica sol | Acidic | Acidic | Alkaline | Plkaline | Alkaline | Acidic | Acidic | Acidic |
| $BaSO_4$/$SiO_2$ mass ratio in slurry to be spray-dried | | 85/15 | 93/7 | 85/15 | 85/15 | 85/15 | 90/10 | 90/10 | 90/10 |

EP 4 495 069 A1

| Item | | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|---|
| Addition of sodium sulfate | Added amount (to BaSO4) (ppm) | - | - | - | - | - | 10 | 100 | 10000 |
| Drying method | | Spray drying | Spray drying | Spray drying | Spray drying | Spray drying | Spray drying | Spray drying | Spray drying |
| Firing temperature x Duration (°C x h) | | 900 × 2 | 900 × 2 | 900 × 2 | 750 × 2 | 900 × 2 | 900 × 2 | 900 × 2 | 900 × 2 |
| Powder physical properties | Average particle size ($\mu$m) | 3.4 | 3.3 | 3.4 | 3.4 | 3.4 | 3.4 | 3.4 | 3.4 |
| | SiO$_2$ content (%) | 15 | 7 | 15 | 15 | 15 | 10 | 10 | 10 |
| | Sphericity | 1.05 | 103 | 104 | 1.07 | 125 | 1.05 | 104 | 107 |
| | Texture | ○ | ○ | ○ | △ | × | ○ | ○ | ○ |

EP 4 495 069 A1

| Item | | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|---|
| Result of test of "barium sulfate" in The Japanese Standards of Quasi-Drug Ingredients 2021 | Phosphate | Not detected | Not detected | Not detected | Not detected | Not detected | Not detected | Not detected | Not detected |
| | Sulfide | Not detected | Not detected | Not detected | Detected | Not detected | Not detected | Not detected | Not detected |
| | Hydrochloric acid-soluble matter (mg) | 17.2 | 7.8 | 19.4 | 21.3 | 42.3 | 8.0 | 8.1 | 275 |
| | Soluble barium salt | Detected | Detected | Not detected | Not detected | Detected | Detected | Detected | Not detected |
| | pH | Neutral | Neutral | Neutral | Neutral | Neutral | Neutral | Neutral | Neutral |
| | Heavy metal (ppm) | 10 or less | 10 or less | 10 or less | 10 or less | 10 or less | 10 or less | 10 or less | 10 or less |
| | Arsenic (ppm) | 1 or less | 1 or less | 1 or less | 1 or less | 1 or less | 1 or less | 1 or less | 1 or less |
| | Satisfied/Not satisfied | Not satisfied | Not satisfied | Not satisfied | Not satisfied | Not satisfied | Not satisfied | Not satisfied | Not satisfied |
| | Absorbance of test liquid after analysis of soluble barium salt (Wavelength 500 nm, Optical path length 20 mm) | 0.936 | 0.853 | 0.016 | 0.015 | 0.032 | 0.916 | 0.425 | 0.007 |

The results in Examples 1 to 8 and Comparative Examples 1 to 8 demonstrate that the method of the present invention enables production of spherical composite particles of barium sulfate and silica satisfying the requirements concerning "hydrochloric acid-soluble matter" and "soluble barium salt" described in the "Barium sulfate" part in "The Japanese Standards of Quasi-Drug Ingredients 2021". The results also demonstrate that all the spherical composite particles of barium sulfate and silica produced by the method have good powder physical properties.

**Claims**

1.  Spherical composite particles of barium sulfate and silica with the following features:

    when the spherical composite particles are subjected to a purity test concerning "hydrochloric acid-soluble matter and soluble barium salt" described in the standard of "Barium sulfate" in "The Japanese Standards of Quasi-Drug Ingredients 2021", the spherical composite particles contain a hydrochloric acid-soluble matter in an amount of 15 mg or less, and
    a test liquid used in the test concerning the "soluble barium salt" has an absorbance of less than 0.03.

2.  The spherical composite particles of barium sulfate and silica according to claim 1,
    wherein the spherical composite particles satisfy the standard of "Barium sulfate" in "The Japanese Standards of Quasi-Drug Ingredients 2021".

3.  The spherical composite particles of barium sulfate and silica according to claim 1 or 2,
    wherein the spherical composite particles have a sphericity of 1.10 or less.

4.  A cosmetic product, comprising the spherical composite particles of barium sulfate and silica according to any one of claims 1 to 3.

5.  A method for producing spherical composite particles of barium sulfate and silica, the production method comprising:

    a first step of washing barium sulfate obtained by a reaction between barium sulfide and sulfuric acid with an aqueous solution of a sulfate of a Group 1 or Group 2 element in the periodic table or with sulfuric acid,
    a second step of repulping the washed barium sulfate in a solvent to prepare barium sulfate slurry,
    a third step involving: mixing the barium sulfate slurry with silica to give a mixture; spray drying a portion of the mixture; firing the dried mixture at 800°C to 1100°C; and subjecting the fired product to a purity test concerning "hydrochloric acid-soluble matter and soluble barium salt" described in the standard of "Barium sulfate" in "The Japanese Standards of Quasi-Drug Ingredients 2021" to obtain a mixture having the following feature (a) or (b),

    (a) the mixture contains a hydrochloric acid-soluble matter in an amount of 15 mg or less, and a test liquid used in the test concerning "soluble barium salt" has an absorbance of less than 0.03, or
    (b) the mixture contains a hydrochloric acid-soluble matter in an amount of 14 mg or less, and a test liquid used in the test concerning "soluble barium salt" has an absorbance of 0.03 or more;

    a fourth step, which is performed when the mixture obtained in the third step has the feature (b), of adding a salt of a Group 1 or Group 2 element in the periodic table to the mixture at a percentage of 0.05 to 0.40% by mass relative to the weight of the raw material barium sulfate;
    a fifth step of spray drying the mixture having undergone or not undergone the addition of a salt of a Group 1 or Group 2 element in the periodic table to obtain a dried product, and
    a sixth step of firing the dried product at 800°C to 1100°C.

6.  The method for producing spherical composite particles of barium sulfate and silica according to claim 5,
    wherein the mixing the barium sulfate slurry with silica in the third step is performed while mixing the silica such that the weight percentage of the silica is 1% or more in the total weight of the barium sulfate and the silica in the barium sulfate slurry.

7.  The method for producing spherical composite particles of barium sulfate and silica according to claim 5 or 6,
    wherein the silica has an average particle size of 1 to 100 nm.

FIG. 1

## INTERNATIONAL SEARCH REPORT

International application No.

**PCT/JP2023/010212**

### A.  CLASSIFICATION OF SUBJECT MATTER

*C01B 33/18*(2006.01)i; *C01F 11/46*(2006.01)i; *A61K 8/23*(2006.01)i; *A61K 8/25*(2006.01)i
FI:   A61K8/25; A61K8/23; C01B33/18 E; C01F11/46 A

According to International Patent Classification (IPC) or to both national classification and IPC

### B.  FIELDS SEARCHED

Minimum documentation searched (classification system followed by classification symbols)

C01B33/18; C01F11/46; A61K8/00-8/99

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

### C.  DOCUMENTS CONSIDERED TO BE RELEVANT

| Category* | Citation of document, with indication, where appropriate, of the relevant passages | Relevant to claim No. |
|---|---|---|
| Y | JP 2018-140921 A (SAKAI CHEMICAL INDUSTRY CO., LTD.) 13 September 2018 (2018-09-13)<br>claims, paragraphs [0020], [0042], [0050]-[0073] | 1-4 |
| A | | 5-7 |
| Y | JP 2016-199454 A (SAKAI CHEMICAL INDUSTRY CO., LTD.) 01 December 2016 (2016-12-01)<br>claims, paragraphs [0004], [0025], [0026], [0028], [0045] | 1-4 |
| A | | 5-7 |
| A | JP 2020-2099 A (MATSUMOTO TRADING CO., LTD.) 09 January 2020 (2020-01-09)<br>paragraph [0028] | 1-7 |
| A | JP 4-309566 A (KAO CORP.) 02 November 1992 (1992-11-02) | 1-7 |
| A | JP 59-122554 A (ONAHAMA SAKAI KAGAKU K.K.) 16 July 1984 (1984-07-16) | 1-7 |

☐ Further documents are listed in the continuation of Box C.     ☑ See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **06 April 2023** | **25 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

<table>
<tr><td colspan="2" align="center">INTERNATIONAL SEARCH REPORT<br>Information on patent family members</td><td colspan="2">International application No.<br><br><b>PCT/JP2023/010212</b></td></tr>
</table>

| Patent document cited in search report | | Publication date (day/month/year) | Patent family member(s) | Publication date (day/month/year) |
|---|---|---|---|---|
| JP 2018-140921 | A | 13 September 2018 | US 2019/0375652 A1<br>claims, paragraphs [0035],<br>[0066], [0081]-[0121]<br>WO 2018/155185 A1<br>EP 3587349 A1<br>CN 110325477 A<br>KR 10-2019-0124212 A | |
| JP 2016-199454 | A | 01 December 2016 | (Family: none) | |
| JP 2020-2099 | A | 09 January 2020 | (Family: none) | |
| JP 4-309566 | A | 02 November 1992 | (Family: none) | |
| JP 59-122554 | A | 16 July 1984 | US 4505755 A<br>US 4551497 A<br>GB 2134094 A<br>GB 2174999 A<br>DE 3347191 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6390756 B

**Non-patent literature cited in the description**

- *The Japanese Standards of Quasi-Drug Ingredients,* 2021